# EUROPEAN PATENT SPECIFICATION

(11) **EP 4 266 987 B1**
(45) Date of publication and mention of the grant of the patent: **26.11.2025**
(21) Application number: 21911654.8
(22) Date of filing: 15.12.2021
(51) Int. Cl.: A61B 5/00, A61B 5/026, A61B 17/11, A61B 17/122, A61B 17/28, A61B 5/0265

(54) **A DEVICE FOR INTRAOPERATIVE MEASUREMENTS OF THE GASTROINTESTINAL TRACT ORGANS' TISSUE PERFUSION**
VORRICHTUNG FÜR INTRAOPERATIVE MESSUNGEN DER GEWEBEPERFUSION VON MAGEN-DARM-TRAKTORGANEN
DISPOSITIF DE MESURES INTRA-OPÉRATOIRES DE PERFUSION DU TISSU D'ORGANES DU TRACTUS GASTRO-INTESTINAL

(30) Priority: 22.12.2020 PL 43645620
(43) Date of publication of application: 01.11.2023
(73) Proprietor: Gdanski Uniwersytet Medyczny, 80-210 Gdansk (PL); Politechnika Gdanska, 80-233 Gdansk (PL)
(72) Inventor: ZIELINSKI, Jacek, 83-110 Tczew (PL); BUJNOWSKI, Adam, 80-180 Jankowo (PL); WTOREK, Jerzy, 80-180 Jankowo (PL); NEUMAN, Tomasz, 80-125 Gdansk (PL)
(74) Representative: JD&P Patent Attorneys Joanna Dargiewicz & Partners
(86) International application number: PCT/PL2021/050087
(87) International publication number: WO 2022/139611

(56) References cited:
- WO-A1-2013/006053
- WO-A1-2013/134411
- WO-A1-2014/153428
- AU-A1- 2018 200 847
- CN-A- 111 134 737
- CN-U- 211 704 740
- DE-A1- 2 709 984
- US-A1- 2018 078 260
- US-B1- 10 806 457

## Description

### Field of invention

The object of the invention is a device for taking intraoperative measurements of the gastrointestinal tract organs' tissue perfusion, particularly before anastomosis needed following resection of a disease-affected fragment of the gastrointestinal tract during a surgery and use thereof.

In particular, the device serves assessing perfusion in such fragments of the gastrointestinal tract as the small intestine, large intestine, oesophagus, and fragments of the stomach.

Correct assessment of tissue perfusion is of significance for elimination of potential postoperative complications. Anastomosis of the gastrointestinal tract following resection must be preceded with an analysis of blood supply to the fragments subject to anastomosis. It is important that the blood supply assessment procedure be reliable but not absorbing and possibly not time consuming. Moreover, should the need arise to change the anastomosed part arise, it should be possible to repeat it.

Considering the spatial measurement properties, it is required that the whole volume of the examined organ fragment be assessed, not only e.g. its surface.

In the event the tissues' perfusion is estimated wrong, and in effect they are anastomosed inappropriately, say the intestine, postoperative complications may develop which, in the best scenario necessitate a corrective surgery, and in the worst variant can lead to serious effects, the patient's death included.

### Prior art

Today, intraoperative examination of blood supply to tissues consists prevailingly in the surgeon's subjective assessment. Assessed is the colour of the tissue fragments of the organs to be anastomosed and the way their open fragments bleed, and on that basis the surgeon (operator) makes the decision on the selection of the tissue areas to be fused.

Also known is another, potentially more objective method of examining tissue perfusion, which consists in injecting a fluorescent marker (contrast) into blood and observing the selected fragment of the organ, e.g. the intestine, in ultraviolet light. An example of the solution was described in application WO2018104552 A1 which presents the method of processing and assessing the hemodynamic image in the anatomical structure of a subject. The method is, however, viewed as troublesome in application and extending the duration of the surgical operation performed under the open method and in minimally-invasive techniques (laparoscopy, robotic surgery). The contrast injected into the circulatory system needs time to show in the area of interest. Furthermore, to observe it, it is necessary to switch off the lights in the operating theatre or, alternatively, use suitable lighting, which makes intraoperative observation of the patient more difficult. In addition, the time before taking another measurement is relatively long, as it is determined by the time constant of the marker decay in blood. The number of measurements repeated is also limited for various reasons. Not insignificant either is the fact that the method is a way of assessing the surface of the organ, i.e. does not enable assessing the whole volume of the considered organ fragment.

Known in prior art too, are methods consisting in measuring changes in the intensity of the light reflected or transmitted by tissues, which are based primarily on different light absorption by oxygenated and non-oxygenated blood. Characteristic for tissue supplied with blood is change in the volume of oxygenated blood synchronic with heartbeat. Measurements taken for several selected light wavelengths, under both the reflection and transmission methods, enable assessing the local blood supply to tissues. With the number of wavelengths selected appropriately, i.e. waves of different, but adequately selected lengths, it is also possible to assess the level of oxygen in blood (commonly called oxygenation). An example of the solution was described in application WO2018236815 A1 which presents an optical sensor or set of optical sensors placed on the surface of various penetrators (catheters) enabling examination of body cavities, e.g. the gastrointestinal system, of various cross-sectional areas. Solutions employing laser Doppler sensors must be classified in the same category of optical devices for measuring blood flow. A solution of the kind was presented in application WO2013149264 A1 which discloses a device for measuring blood perfusion in the oesophagus tissue. Thanks to the fact that the system is equipped with a specially built catheter, the pressure is measured in the area of the oesophagus monitored for blood flow, as well as reflux episodes by way of measuring changes in electrical impedance in the space surrounding the sensor. A similar solution was presented in the description of patent RU2406444 C1 which discloses a method of diagnosing and assessing blood flow in the inferior trochanteric artery in cases of colon cancer, and applies in particular to blood supply to the inferior section of the transplant in cancerous patients during intraoperative examinations, where it is characterized in that during the surgery a Doppler USG image of the inferior trochanteric artery is performed and a cross-section image thereof is taken. The preliminary tests are conducted in the mode of colour Doppler flow mapping, followed by spectra analysis of the blood flow in the pulsed wave Doppler mode where assessment of the frequency shift spectra serves identifying the qualitative and quantitative parameters of blood flow. The description of patent RU2471428 C1, on the other hand, discloses the way of assessing microcirculation in the mucous membrane of the large intestine in the area of blood supply to the superior and inferior mesenteric arteries under methods utilizing laser Doppler sensors and optical oximetry of tissues. The measurements serve identification of the microcirculation indicators, functional blood oxyhemoglobin saturation in the vascular bed, and relative volumes of all hemoglobin fractions in the tested tissue volume sampled endoscopically during colonoscopy.

Known too, is the way of measuring tissue volume changes triggered by pulsatile blood flow. To monitor the changes, the sensor incorporates a flexible element the diameter of which is selected so that its wall remains in direct contact with the tested tissue, e.g. the intestine wall. The changing tissue volume causes measurable changes of pressure inside the flexible element. An example of the solution was presented in WO2006088355 A1. A similar idea was used in EP1246562 B1 to measure blood pressure in the portal vein.

Assessment of blood supply to tissues can also be attained by measuring changes in their electrical impedance caused by changing blood volume, provided that the measurements are taken for one selected frequency out of the range of 20-100 kHz. The minor changes in the impedance are synchronous with the mechanical heartbeat (i.e. with the ECG signal too) and testify to fluctuations of the volume of the blood pulsating in the tested tissue area.

The method presented above is built upon in impedance spectroscopy which consists in measuring changes in the impedance and the impedance of the tissue itself for a specified, broader range of frequencies or several discrete frequencies out of the range. Thanks to the measurements of the impedance modulus and phase for different, appropriately selected frequencies, it is also possible to differentiate between live tissue necrotic tissue.

Except for the fluorescence technique, the others listed above have not so far been applied to any broader extent in assessing blood supply in the gastrointestinal tract. The cause thereof lies in non-existence of appropriate measurement procedures or adequate modification of the sensors, which could ensure the necessary measuring properties of the application.

Known in prior art too are devices for intraoperative testing of blood supply to tissues. For instance, the description of invention DE2709984 A1 discloses a probe for measuring the internal pressure in blood vessels or the intestines, where the probe has a measuring head coated with a cured, two-component sleeve of silicone rubber and where in effect of appropriate selection of the elasticity coefficient two separate parts are identified working as elements which move in response to the pressure in the surrounding and tested medium, each of which is mounted with two plate springs fitted with induction cores with each of the ferromagnetic core capable of moving with respect to the head of the induction coil placed inside to generate a change in its inductance, the latter measured with a proper electronic measuring system. The device characterized in that it is highly sensitive and its response time is short.

US6216024 B1 provides a method and device to assess circulatory impairment in a patient, for example in perfusion insufficiency, through measuring pCO2 in the patient's upper alimentary tract and/or respiratory tract. The carbon dioxide sensor can be placed in the patient's mouth or nose and does not require inserting the carbon dioxide sensor in the upper section of the gastrointestinal tract. Avoiding passage through the mouth to the throat and oesophagus one avoids discomfort and minimizes the risk of injury. The device according to the invention is composed of a carbon dioxide sensor, sensor which measures the pCO₂ level as the result of its contact with the mucosa, and an element isolating the area of the mucosa used in the measurements from the air in the surrounding area, and resources enabling determination of the degree of perfusion insufficiency in the patent, resulting from the detected partial pressure of carbon dioxide. WO 2013/006053 A1 discloses a system and a method for predicting the viability of a body tissue in a patient.

Considering the above listed determinants and limitations, one can conclude that there is the need to provide a device for swift and objective assessment of blood supply to the fragments of the organs subject to anastomosis, which should be easy to use and at the same time take reliable measurement which does not affect the surgery time.

### Detailed Description of the Invention

The invention provides a device for intraoperative assessment of blood supply to the tissue perfusion of the gastrointestinal tract, as defined by claim 1, to measure the blood supply to the organs of the gastrointestinal tract during surgical operations, especially the anastomosed tubular parts of the gastrointestinal tract such as the small intestine, large intestine, oesophagus, and fragments of the stomach.

When taking measurements with the device according to the invention, one makes use of two pressure actuators in the adjacent areas, and a set of combined sensors arranged as appropriate on the mechanical body of the measuring probe which encases the part of the organ to be anastomosed, where the sensors react to changes in various parameters, including the blood supply to the tissue.

The device uses the results of simultaneous measurements of several parameters, obtained under different techniques (modalities) and their dependence on temporary blood level which changes in effect of the maneuvers performed by the pressure actuators.

The two-stage measurement taking takes place in controlled pressure applied to two areas of the tested tissue. In stage one, pressure is applied resulting to the closure of the blood vessels through which blood can flow out and which were severed as the result of severing the disease-affected part together with the adjacent disease-free part. The vessels run in the area where the morbid tissue of the organ, e.g. the intestine, has been resected.

Simultaneously measured is the change in the parameters of the tissue adjacent on the area with the combined sensors (possible are combinations of various signals measured: optical - for several light wavelengths, impedance - of one and multiple frequencies, magnetic - caused by changeable flow of magnetized blood, temperature - connected with heating and/or cooling, ultrasound, etc.) as of the moment the blood outflow from the circulation system through vessels severed in surgery is stopped.

The moment the values of the parameters of the signals measured stabilize, the second stage of assessment begins. Pressure is applied using the second pressure actuator to the tissue adjacent on the area which has been subject to pressure preventing outflow of blood from the circulation system, where the sensors are applied so that whenever necessary some of the elements are placed outside, the others inside the tested intestine.

The pressure build-up speed in the second actuator, i.e. in the tested tissue itself, is selected so that over a single time between heart beats the pressure can be deemed constant. This is achievable e.g. by controlling pressure changes with the electrocardiograph signal. Electrocardiogram is also used as the reference signal to identify the delay of the pulse wave spreading in the vascular tree of the assessed tissue of the gastrointestinal tract versus the stimulation which triggers heart contraction measured indirectly with the combined sensors.

Simultaneously with the measurement-taking, the changes in the parameter values is analyzed and whenever a pathological state is indicated (no sufficient blood supply) expanded measurements are taken, e.g. impedance measurements are replaced with impedance spectroscopy.

The vitality of the tissue in the areas between the elements of each combined sensor is identified based on an analysis of the accumulated signals in the two measurement phases. The algorithm of analyzing the signals and assessing blood supply and vitality of the tissue is run by a microcontroller of the device and can be supported with calculations performed on a parent computer fed with data during the measurements via a link, e.g. the radio. The tasks of the microcontroller also include automating the measurement-taking procedure.

The object of the invention is a device for intraoperative measurements of the gastrointestinal tract organs' tissue perfusion during theirs surgical anastomosis, characterized in that it contains a holder 5 composed of two arms crossing over at the connection point, where the first and the second ends of both arms: upper arm 5a and lower arm 5b are arranged parallel so that on the same, mutually parallel ends the lower arm 5b is fitted with an internal sleeve 2 and external sleeve 8, with measuring sensors 4 arranged along the outer circumference of the internal sleeve 2 and on the inner circumference of the external sleeve 8, where on its inner circumference the external sleeve 8 has a groove accommodating a flexible pressing conduit in the form of a torus 3 which is fillable with a medium and enables the closure of the clearance between the sleeves 2 and 8 and in this way enables downthrusting a tissue 1 placed therein, wherein the torus 3 is connected to a pump Pump 2 and a valve Valve 2 to enable the filing it with a medium and controlling of pressure inside the torus 3, and the upper arm 5a is fitted with pressing elements 6 and 9, wherein at its first ends each of the arms 5a and 5b has at least one measuring sensor 7, wherein the sensors 7 are located opposite each other.

In the device according to the invention, in the area of first ends of arms 5a and 5b the top arm 5a is fitted with a pressing element 10, whereas in the area of the second ends of arms 5a and 5b the top arm 5a is fitted with element 11.

Preferably, in the device the first end of the top arm 5a is cylinder shaped and ending with a pressing element 10 in its top part, and contains inside the pressing element 6 which in the lower part is shaped to form a piston, wherein the pressing element 6 fixed on the first end of the top arm 5a serves regulation of the pressure and the downthrusting of the tested tissue 1 to the measuring sensors 7, and the force of its downthrust is regulated by the pressure of the medium supplied by the pressing element 9.

Preferably, in the device element 11 is shaped to form a screw regulating the distance between the second ends of arms 5a and 5b of the holder 5. Element 11 limits the maximum squeezing force exerted on the tissue 1 mechanically, where this is attained by the holder 5.

Preferably, in the device the measuring sensors 4 are located at the place where the tested tissue 1 is squeezed by the torus 3.

Preferably, in the device the sensors 7 contain a measuring and control system which includes an MCU microcontroller to which a measuring set for taking measurements of electrical impedance dZ1 - dZn is connected via an MUX1 multiplexer and to which a measurement-taking set for optical measurements PPG1- PPGn is connected via an MUX2 multiplexer, where the MCU microcontroller is additionally connected to the ECG electrocardiogram measurement-taking unit, a power supply battery Battery, a radio forming a link Radio, a data display Displ, pressure gauges MCisn1, MCisn2, pumps Pump1, Pump2 forcing pressure in the actuators correspondingly in torus 3 and pressing element 6 via Cuff1 and Cuff 2, and electric valves Valve 1, Valve 2 which enable releasing air from the measuring system; moreover, Pump 2 and Valve2 are connected to Cuff 2, and Cuff 2 is connected to the pressure gauge MCisn2.

Preferably, in the device the measuring sensor 7 serves taking simultaneous measurements of optical transmittance and electrical impedance in the tested organ tissue under controller pressure.

The described device can be used for measuring blood supply to the organs of the gastrointestinal tract during surgical operations, especially the anastomosed tubular parts of the gastrointestinal tract such as the small intestine, large intestine, oesophagus, and fragments of the stomach.

Preferably, the device consists in fusing the ends of the examined tissue 1 positioned in between the inner sleeve 2 and outer sleeve 8, whereupon the tissue 1 is pressed against the inner sleeve 2 with a flexible, pressing conduit 3 formed in the shape of a torus which is placed in the outer sleeve 2 and filled with air whilst the air pressure is being controlled; then, using the combined measuring sensors 7 the measurements are taken under controlled pressure of the sensors on the tissue 1, where the measuring sensors 7 build up pressure in the volume of the tissue 1 within their range to the value maximizing the pulsation of the arteries triggered by pulse propagation.

Preferably, the impedance and light absorption are measured by measuring sensors 7 which enable taking the measurements of the tissue optical transmittance for selected optical wavelengths and simultaneous measurements of the electrical impedance for several frequencies of the current, provided that in the event changes are discovered indicating reduced blood supply the procedure is extended by taking spectroscopic impedance measurements with the use of the same measuring probes 4 on the controlling element.

### Description of the Figures

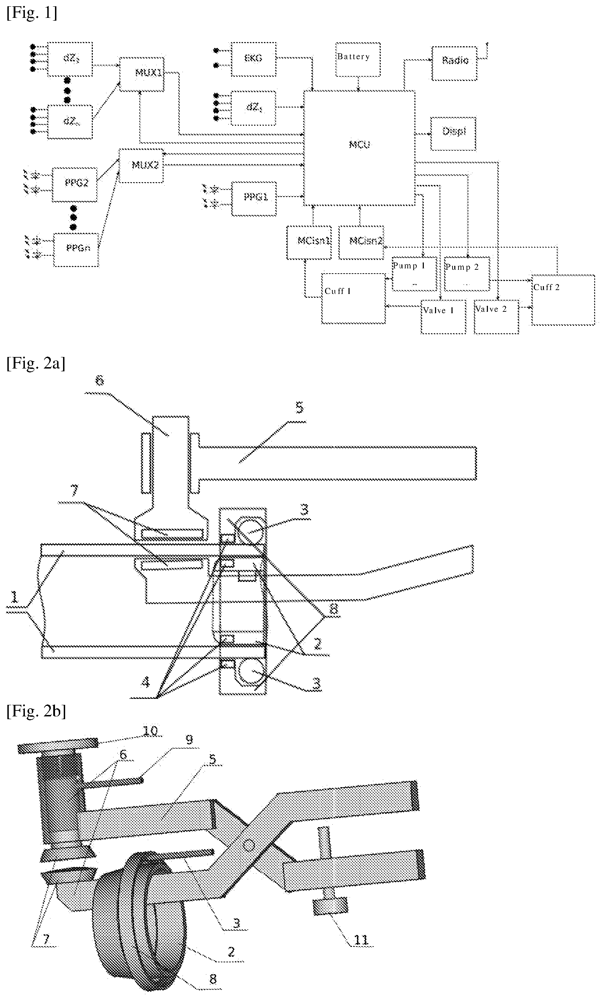

[Fig.1] presents a block diagram of the measurement and control system.
   List of symbols:
   MCU - microcontroller;
   Displ - display;
   PPG1 - PPGn - optical measurements system;
   dZ1 - dZn - electrical impedance measurements system;
   MUX1 - MUX2 - multiplexer;
   EKG - electrocardiogram measurement system;
   Radio - radio link;
   Battery - power supply;
   MCisn1 - MCisn2 - pressure gauge;
   Pump 1- Pump 2 - pumps building up pressure in the actuators;
   Valve 1- Valve 2- electric valves enabling the release of air from the system
[Fig. 2a] presents a cross-section of the device in one of the variants, with one measuring sensor (7).
[Fig. 2b] presents a general view of the device in one of the variants, with one measuring sensor (7).
[Fig. 3a] presents a general view of the device in one of the variants, with many measuring sensors (7).

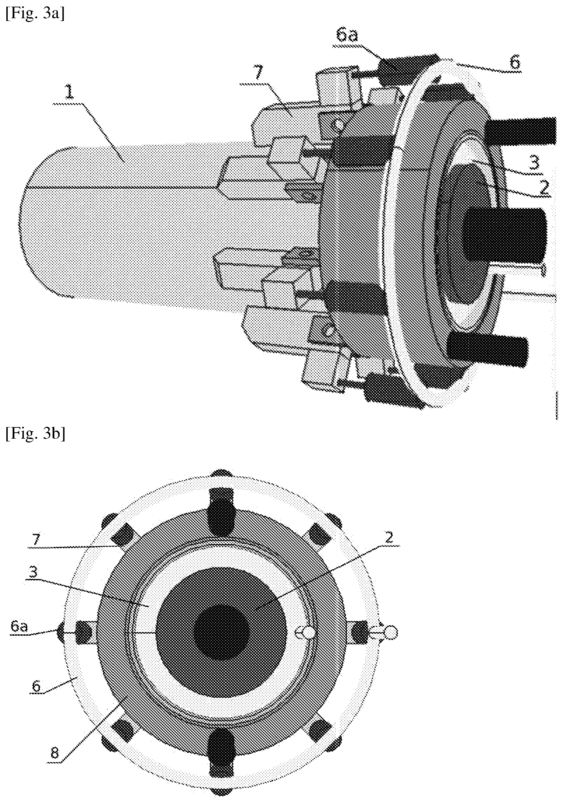

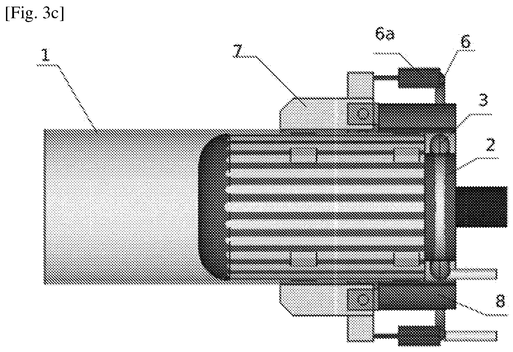

[Fig.3 b] presents a general view of the device in one of the variants, with many measuring sensors (7)
[Fig.3 c] presents a side view of the device in one of the variants, with two measuring sensors (7)

List of numerical references:
1 - a fragment of the tested tissue - e.g. small intestine;
2 - the internal sleeve in the form of a cylinder shell continuing to become the shell of a truncated cone, the heights of both surfaces selected as appropriate;
3 - the flexible pressing conduit in the form of a torus - which presses on the end of the intestine when filled with medium (air, fluid) at proper pressure - depending on the measurement configuration - downthrust from inside to the external sleeve or from outside to the internal sleeve (embodiment 1);
4 - the sensor, or composite sensor to take reference measurement at the point where the resected tissue is compressed;
5 - the arm of the holder enabling the holding of the device (embodiment 1)
6 - the pressing element in embodiment 1; in embodiment 2 the pressing elements are 6 and 6a and play the same function - they ensure controlling the pressure force of measuring electrodes 4 to the tissue;
7 - the sensor or composite sensor - used in embodiment 1 is one sensor, whereas in embodiment 2 the number of sensors is multiple;
8 - the external sleeve with the groove accommodating the flexible conduit (3) which enables closure of the clearance in between the sleeves (2) and (8) and in this way enables downthrusting the tissue placed therein;
9 - the element pressing the measuring sensor or its element to the tissue (in embodiment 1); together with 6 - it plays the same function as 6 and 6a in embodiment 2;
10 - the mechanism serving preliminary pressure regulation (the sensor's pressing force on the tissue) only in embodiment 1.
11 - the element limiting the maximum pressure force, only in embodiment 1.

### Experimental part

Irrespective of embodiment variant, the device is made up of a measurement and control system, pressure generation system, and measuring probes. Depending on the envisaged application and execution, the measuring probes may differ between themselves in terms of e.g. the number of the measuring channels, the number and arrangement of the sensors (optical, impedance, magnetic, and any other the use of which is possible) also with respect to one another, e.g. they may be arranged alternately or composed. Measurement probes may be directly integrated with the measurement and control system and the pressure generation system, or may simply be connected using an appropriate bundle of electric wires and pneumatic lines. The way in which blood supply is assessed, however, and hence the working principle is identical in all cases.

The device for intraoperative measurements of tissue perfusion consists of an electronic measurement and control system (Fig.1) and a measuring probe. The measurement and control system is a dedicated system for taking simultaneous, generally multichannel measurements using sensors which enable assessment of the changes of the optical, impedance, magnetic, thermal, ultrasound, and other properties.

In the presented case, the measurement and control system incorporates: dZ1 ÷ dZn - a system (measuring unit) for measuring electric impedance connected to a microcontroller unit (MCU) via an MUX1 multiplexer; PPG1 ÷ PPGn - a system (measuring unit) for taking optical and/or magnetic and/or thermal measurements, connected to an MCU via an MUX2 multiplexer. In addition, the MCU (microcontroller) is connected to the system of the electrocardiogram (ECG - measuring unit), power supply battery, link preferably of the wireless type, Displ data display, pressure gauges MCisn1-2, and two air pumps Pump 1-Pump 2, and two valves Valve 1- Valve 2.

The sensors shown in the presented examples are of the optical and impedance type, but they may be replaced or integrated with magnetic, thermal, ultrasound, and other sensors.

### Example 1 (variant 1)

The embodiment of the measuring device (Fig. 2a, Fig.2b) may be composed of one composite sensor and one reference sensor, or according to the embodiment shown in (Fig. 3 a, Fig.3b) of a larger number of composite sensors arranged around the circumference. The device enables invoking different and simultaneously controller pressure levels in the tissue placed in the measuring probe.

The device is made up of one or a larger number of measuring sensors (7) which enable simultaneous measurements of optical transmittance and electric impedance in the conditions of controlled pressure in the examined tissue.

Measurement of vitality of the organs of the gastrointestinal tract in the course of a surgery (intraoperative examination) was presented on the example of examination of small intestine tissues. In the course of resection blood flow from the examined end of gastrointestinal tract to the surrounding area is stopped by applying temporary pressure to the blood vessels which enable such blood flow caused by resecting a section of gastrointestinal tract. The anastomosed ends of the intestine (1), are, one after another, placed between the internal sleeve (2) and external sleeve (8). The elements play the role of a stabilizer. The clamping of the tissue of the intestine end (1) is effected by pressing it against the internal sleeve (2) or external sleeve (8) with an elastic conduit in the shape of a compressing torus (3) which is filled with air or fluid while controlling the pressure inside the torus (3). Element (11) limits the maximum strength of clamping the tissue which can be mechanically exerted with a holder (5). In addition, the element (10) enables preliminary regulation of pressure and compression of the tissue against the measuring sensors (7) using the pressing element (6) and the piston inside it, where its adherence force is regulated by the pressure of air/fluid supplied by (9).

During the measurements, a tissue fragment is placed between the elements of the sensor/ composite measuring sensor (7). Depending on the composite sensor type(s) measured is the parameter corresponding to the sensor type and its changes in time (e.g. electric impedance, optical wave absorption, etc.) using sensors and the measurement and control system. The measurements are taken at a known value of pressure caused by way of the pressing element which presses the measuring sensor (7) against the measured tissue. In addition, the pressure is monitored and appropriately modified. The pressures, optical wave absorption, electric impedance, etc. are measured with measuring units and appropriate elements arranged on the internal sleeve (2) and external sleeve (8). For example, the measuring electrodes (4) used for impedance measurements are placed on both sleeves: (2) and (8).

The electric impedance measuring units dZ1-.dZn are multiplexed using multiplexer MUX1. In addition, measurements of the tissue's optical transmittance are taken by the measuring unit PPG1 directly at the compressed fragment of the intestine and by the measuring units PPG2-PPGn multiplexed using MUX2 are taken in the examined and assessed area. The pressure is measured and monitored using pressure gauges MCisn₁₋₂. All measurements are taken simultaneously to the measurements of the ECG signal. The microcontroller (MCU) analyses the signals and sends the processed data to the superior system via a low power radio link (Radio). In addition, the results of an analysis of the processed signals performed using an MCU or an external system may be displayed directly in the device on the provided display. The probe, the measuring system included, is powered with batteries.

Used as a reference signal is electrocardiogram of the operated patient (person). The measuring sensors in the volume of tissue within them generate pressure in it with a value that maximizes the pulsations of the arteries caused by the propagation of the pulse. The sensors enable taking measurements of the tissue optical transmittance for selected lengths of the optical wave together with simultaneous measurements of electric impedance for a number of current frequencies which allow monitoring of the cellular tissue structure. Once the blood flow from the vascular tree of the examined part of the gastrointestinal tract is closed, monitored is the increase in the volume of the flowing blood using both measuring techniques (impedance and optical measurement). Once the vascular tree is filled with blood to the maximum, pressure is generated in the tissue so as to maximize arterial pressure.

### Example 2 (variant 2)

Measurements of the examined tissue of small intestine are taken by placing each end of the intestine (1) between the internal sleeve (2) and external sleeve (8). The elements play the role of a stabilizer. The resected end of the intestine (1) is constricted using an elastic conduit filled with compressed air (torus) (3), which - when inflating - locks the intestine (1) in place between the internal sleeve element (2) and the external sleeve (8). The measuring electrodes are found partially on the internal sleeve (2) and the combined sensor (7) which is pressed against the intestine (1) with controlled force by a piston system (6a) filled with compressed gas supplied via the pressing element (6). The pressure of the gas (air) is regulated so as to enable measuring the parameters of blood supply to the intestine. The solution features several sets of composite sensors (preferably eight) arranged at even distances on a fragment of the intestine (1). It enables swifter assessment of blood supply to the tissues.

Examples of working parameters for invention embodiments.
1. Range of pressure values which cut off blood supply ≤ 300 mmHg
2. Pressure resulting in the pressing of composite measuring sensors 30 ÷ 150 mmHg
3. Application current to measure the impedance 0.01 ÷ 0.5 mA
3. Range of measurement frequencies 5 ÷ 5000 kHz
4. Range of the measured impedance (module) 0 ÷ 500 Ω
5. Resolution of the measurement of impedance changes 10 mΩ
6. Light wavelength ranges 0.660 ÷ 2 µm
7. Measured temperature range 20 ÷ 40°C
8. Resolution of the measurement of temperature changes 0.05°C
9. Range of the measured magnetic field nT ÷ mT
10. Range of frequencies of the measured ultrasound signals ≤ 20 MHz

## Claims

1. A device for taking an intraoperative measurements of the gastrointestinal tract organs' tissues perfusion during their surgical anastomosis containing: a holder (5) composed of two arms crossing over at a connection point, where both ends - first and second ones - of both arms - an upper arm (5a) and a lower arm (5b) - are arranged parallel to each other so that on the same, mutually parallel ends, the lower arm (5b) is fitted with an internal sleeve (2) and an external sleeve (8), with measuring sensors (4) arranged along an outer circumference of the internal sleeve (2) and on an inner circumference of the external sleeve (8), wherein on its inner circumference the external sleeve (8) has a groove accommodating a flexible pressing conduit in the form of a torus (3) which is fillable with a medium and configured to enable closure of a clearance between the internal sleeve (2) and the external sleeve (8), and in this way configured to enable downthrusting a tissue (1) placed therein, wherein the torus (3) is connected to a pump (Pump 2) and a valve (Valve 2) to enable filling it with a medium and controlling of pressure inside the torus (3), and the upper arm (5a) is fitted with pressing elements (6, 9), wherein at its first ends each of the arms (5a and 5b) has at least one measuring sensor (7), and wherein the sensors (7) are located opposite each other.

2. The device according to Claim 1, **characterized in that** in an area of first ends of arms (5a, 5b), the top arm (5a) is fitted with a pressing element (10), whereas in an area of the second ends of arms (5a, 5b), the top arm (5a) is fitted with an element (11).

3. The device according to Claim 2, **characterized in that** the first end of the top arm (5a) is cylinder shaped and ending with the pressing element (10) in its top part, and contains inside the pressing element (6) which in the bottom part is shaped to form a piston, wherein the pressing element (6) fixed on the first end of the top arm (5a) is configured to regulate the pressure applied to the tested tissue (1) against the measuring sensors (7), and force of its pressing force is regulated by pressure of the medium supplied by the pressing element (9).

4. The device according to Claim 2, **characterized in that** the element (11) is shaped to form a screw configured to regulate a distance between the second ends of arms (5a, 5b) of the holder (5).

5. The device according to Claim 1, **characterized in that** the measuring sensors (4) are located at a place where the tested tissue (1) is squeezed by the torus (3).

6. The device according to any of Claims 1-5, **characterized in that** the sensors (7) contain a measuring and control system which includes a microcontroller (MCU), to which a measuring set for taking measurements of electrical impedance (dZ1 - dZn) is connected via a multiplexer (MUX1) and to which a measurement-taking set for optical measurements (PPG1- PPGn) is connected via a multiplexer (MUX2), where the microcontroller (MCU) is additionally connected to the electrocardiogram measurement-taking unit (ECG), a power supply battery (Battery), a radio forming a link (Radio), a data display (Displ), pressure gauges (MCisn1, MCisn2), pumps (Pump1, Pump2) configured to force pressure in the actuators correspondingly in torus (3) and the pressing element (6) via (Cuff1) and (Cuff 2) and electric valves (Valve 1, Valve 2) which enable releasing air from the measuring system, moreover, (Pump 2) and (Valve 2) are connected to (Cuff 2), and (Cuff 2) is connected to the pressure gauge (MCisn2).

7. The device according to Claim 6 **characterized in that** the measuring sensor (7) is configured to take simultaneous measurements of optical transmittance and electrical impedance in the tested organ tissue under controlled pressure.

## Patentansprüche

1. Eine Vorrichtung zur Durchführung einer intraoperativen Messung der Gewebeperfusion der Magen-Darm-Traktorgane während ihrer chirurgischen Anastomose, umfassend einen Halter (5), der aus zwei Armen besteht, die sich an einem Verbindungspunkt kreuzen, wobei beide Enden - erste und zweite - beider Arme - eines oberen Arms (5a) und eines unteren Arms (5b) - parallel zueinander angeordnet sind, so dass an denselben, zueinander parallelen Enden der untere Arm (5b) mit einer Innenhülse (2) und einer Außenhülse (8) ausgestattet ist, mit Messsensoren (4), die entlang eines Außenumfangs der Innenhülse (2) und an einem Innenumfang der Außenhülse (8) angeordnet sind, wobei die Außenhülse (8) an ihrem Innenumfang eine Nut aufweist, die eine flexible Druckleitung in Form eines Torus (3) aufnimmt, der mit einem Medium befüllbar ist und ausgebildet ist, um das Schließen eines Zwischenraums zwischen der Innenhülse (2) und der Außenhülse (8) zu ermöglichen, und auf diese Weise ausgebildet ist, um das Herunterdrücken eines darin platzierten Gewebes (1) zu ermöglichen, wobei der Torus (3) mit einer Pumpe, Pump 2, und einem Ventil, Valve 2, verbunden ist, um das Befüllen mit einem Medium und das Steuern des Drucks innerhalb des Torus (3) zu ermöglichen, und der obere Arm (5a) mit Druckelementen (6, 9) ausgestattet ist, wobei jeder der Arme (5a und 5b) an seinen ersten Enden zumindest einen Messsensor (7) aufweist, und wobei die Sensoren (7) einander gegenüberliegend positioniert sind.

2. Die Vorrichtung gemäß Anspruch 1, **dadurch gekennzeichnet, dass** in einem Bereich der ersten Enden von Armen (5a, 5b) der obere Arm (5a) mit einem Druckelement (10) ausgestattet ist, während in einem Bereich der zweiten Enden von Armen (5a, 5b) der obere Arm (5a) mit einem Element (11) ausgestattet ist.

3. Die Vorrichtung gemäß Anspruch 2, **dadurch gekennzeichnet, dass** das erste Ende des oberen Arms (5a) zylinderförmig ist und mit dem Druckelement (10) in seinem oberen Teil endet und im Inneren das Druckelement (6) enthält, das in dem unteren Teil geformt ist, um einen Kolben zu bilden, wobei das Druckelement (6), das an dem ersten Ende des oberen Arms (5a) befestigt ist, ausgebildet ist, um den Druck, der auf das getestete Gewebe (1) ausgeübt wird, gegen die Messsensoren (7) zu regulieren, und die Kraft seiner Druckkraft durch den Druck des Mediums reguliert wird, das durch das Druckelement (9) zugeführt wird.

4. Die Vorrichtung gemäß Anspruch 2, **dadurch gekennzeichnet, dass** das Element (11) geformt ist, um eine Schraube zu bilden, die ausgebildet ist, um einen Abstand zwischen den zweiten Enden von Armen (5a, 5b) des Halters (5) zu regulieren.

5. Die Vorrichtung gemäß Anspruch 1, **dadurch gekennzeichnet, dass** die Messsensoren (4) an einer Stelle positioniert sind, an der das getestete Gewebe (1) durch den Torus (3) zusammengedrückt wird.

6. Die Vorrichtung gemäß einem der Ansprüche 1-5, **dadurch gekennzeichnet, dass** die Sensoren (7) ein Mess- und Steuersystem enthalten, das einen Mikrocontroller (MCU), umfasst, mit dem ein Messsatz zur Durchführung von Messungen der elektrischen Impedanz (dZ1 - dZn) über einen Multiplexer (MUX1) verbunden ist, und mit dem ein Messdurchführungssatz für optische Messungen (PPG1 - PPGn) über einen Multiplexer (MUX2) verbunden ist, wobei der Mikrocontroller (MCU) zusätzlich mit der Elektrokardiogramm-Messdurchführungseinheit (ECG) einer Stromversorgungsbatterie, Battery, einer Funkvorrichtung, die eine Verbindung bildet, Radio, einer Datenanzeige ( Displ), Druckmessern (MCisn1, MCisn2), Pumpen (Pump1, Pump2) die ausgebildet sind, um Druck in den Aktuatoren entsprechend in dem Torus (3) und dem Druckelement (6) über (Cuff1) und (Cuff2) zu erzwingen, und elektrischen Ventilen (Valve 1, Valve 2) verbunden ist, die das Ablassen von Luft aus dem Messsystem ermöglichen, außerdem (Pump 2) und (Valve 2) mit (Cuff2) verbunden sind, und (Cuff2) mit dem Druckmesser (MCisn2) verbunden ist.

7. Die Vorrichtung gemäß Anspruch 6, **dadurch gekennzeichnet, dass** der Messsensor (7) ausgebildet ist, um gleichzeitige Messungen der optischen Durchlässigkeit und der elektrischen Impedanz in dem getesteten Organgewebe unter kontrolliertem Druck vorzunehmen.

## Revendications

1. Dispositif permettant de mesurer en peropératoire la perfusion des tissus des organes du tractus gastro-intestinal pendant leur anastomose chirurgicale, comprenant un support (5) composé de deux bras se croisant en un point de connexion, où les deux extrémités - première et deuxième - des deux bras - un bras supérieur (5a) et un bras inférieur (5b) - sont disposées parallèlement l'une à l'autre de telle sorte que, sur les mêmes extrémités mutuellement parallèles, le bras inférieur (5b) est muni d'un manchon interne (2) et d'un manchon externe (8), avec des capteurs de mesure (4) disposés le long d'une circonférence extérieure du manchon interne (2) et sur une circonférence intérieure du manchon externe (8), dans lequel, sur sa circonférence intérieure, le manchon externe (8) comporte une rainure recevant un conduit de pression flexible en forme de tore (3) qui peut être rempli d'un fluide et qui est configuré pour permettre la fermeture d'un espace entre le manchon interne (2) et le manchon externe (8) et ainsi configuré pour permettre la poussée vers le bas d'un tissu (1) placé à l'intérieur, dans lequel le tore (3) est relié à une pompe (pompe 2) et à une vanne (Valve 2) pour permettre son remplissage avec un fluide et le contrôle de la pression à l'intérieur du tore (3), et le bras supérieur (5a) est équipé d'éléments de pression (6, 9), dans lequel, à ses premières extrémités, chacun des bras (5a et 5b) comporte au moins un capteur de mesure (7), et dans lequel les capteurs (7) sont situés en face l'un de l'autre.

2. Le dispositif selon la revendication 1, **caractérisé en ce que**, dans une zone des premières extrémités des bras (5a, 5b), le bras supérieur (5a) est équipé d'un élément de pression (10), tandis que dans une zone des secondes extrémités des bras (5a, 5b), le bras supérieur (5a) est équipé d'un élément (11).

3. Le dispositif selon la revendication 2, **caractérisé en ce que** la première extrémité du bras supérieur (5a) est de forme cylindrique et se termine par l'élément de pression (10) dans sa partie supérieure, et contient à l'intérieur l'élément de pression (6) qui, dans la partie inférieure, est formé pour former un piston, dans lequel l'élément de pression (6) fixé sur la première extrémité du bras supérieur (5a) est configuré pour réguler la pression appliquée au tissu testé (1) contre les capteurs de mesure (7), et la force de sa pression est régulée par la pression du fluide fourni par l'élément de pression (9).

4. Le dispositif selon la revendication 2, **caractérisé en ce que** l'élément (11) est formé de manière à constituer une vis configurée pour réguler une distance entre les secondes extrémités des bras (5a, 5b) du support (5).

5. Le dispositif selon la revendication 1, **caractérisé en ce que** les capteurs de mesure (4) sont situés à un endroit où le tissu testé (1) est comprimé par le tore (3).

6. Le dispositif selon l'une quelconque des revendications 1 à 5, **caractérisé en ce que** les capteurs (7) contiennent un système de mesure et de contrôle qui comprend un microcontrôleur (MCU), auquel un ensemble de mesure pour prendre des mesures d'impédance électrique (dZ1 - dZn) est connecté via un multiplexeur (MUX1) et auquel un ensemble de prise de mesures pour des mesures optiques (PPG1- PPGn) est connecté via un multiplexeur (MUX2), le microcontrôleur (MCU) étant en outre connecté à l'unité de prise de mesures d'électrocardiogramme (ECG), à une batterie d'alimentation (Battery), à une radio formant une liaison (Radio), à un affichage de données (Displ), à des manomètres (MCisn1, MCisn2), des pompes (Pump1, Pump2) configurées pour forcer la pression dans les actionneurs de manière correspondante dans le tore (3) et l'élément de pression (6) via (Cuff1) et (Cuff 2) et des vannes électriques (Valve 1, Valve 2) qui permettent de libérer l'air du système de mesure, de plus, (Pump 2) et (Valve 2) sont connectés à (Cuff 2), et (Cuff 2) est connecté au manomètre (MCisn2).

7. Le dispositif selon la revendication 6, **caractérisé en ce que** le capteur de mesure (7) est configuré pour effectuer des mesures simultanées de la transmittance optique et de l'impédance électrique dans le tissu organique testé sous une pression contrôlée.
